(19) Europäisches Patentamt
European Patent Office
Office européen des brevets

(11) Publication number : **0 122 324 B2**

(12) **NEW EUROPEAN PATENT SPECIFICATION**

(45) Date of publication of the new patent specification : **03.02.93 Bulletin 93/05**

(51) Int. Cl.$^5$ : **C07C 233/00,** A61K 7/06, A61K 7/48

(21) Application number : **83111302.2**

(22) Date of filing : **11.11.83**

# (54) Polyquaternary ammonium compounds and cosmetic compositions containing them.

The file contains technical information submitted after the application was filed and not included in this specification

(30) Priority : **15.04.83 US 485197**

(43) Date of publication of application : **24.10.84 Bulletin 84/43**

(45) Publication of the grant of the patent : **07.01.88 Bulletin 88/01**

(45) Mention of the opposition decision : **03.02.93 Bulletin 93/05**

(84) Designated Contracting States : **CH DE FR GB LI NL SE**

(56) References cited :
**FR-A- 7 817 373**
**FR-A-76 029 48**
**GB-A- 2 066 663**
**GB-A-20 666 63**
**US-A- 3 734 889**
**US-A-41 573 88**
**Chemical Abstract 49, 15861h (1955)**

(73) Proprietor : **MIRANOL INC.**
**68 Culver Road**
**Dayton New Jersey (US)**

(72) Inventor : **Desai, Bharat B.**
**5 Sebring Round**
**Belle Mead, NJ (US)**

(74) Representative : **Baillie, Iain Cameron et al**
**c/o Ladas & Parry, Altheimer Eck 2**
**W-8000 München 2 (DE)**

EP 0 122 324 B2

## Description

Background of the invention

The invention relates to scale inhibitors, flocculants and conditioning agents for skin and hair, and processes for their preparation and their use. The compounds of this invention are novel polyquaternary ammonium compounds.

U.S. Patent No. 4,157,388 of Christiansen describes compositions containing polyquaternary ammonium compounds for use as hair conditioners and antistats and humectants for fibrous textile products. These compounds are generally liquid at room temperature. Mirapol® A-15 is a compound in accordance with Examples of Christiansen having the formula:

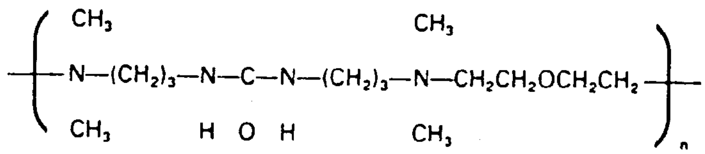

wherein n is at least one. The present polyquaternary ammonium compounds were found to be easier to work with since they do not generally require a particular order of addition and can be added in the oil or water phase. The present compounds in general also are found to have a better water solubility than the prior art compounds of Christiansen.

British Patent Specification GB-A-2066663 describes a variety of polyquaternary compounds and states that they are of use in cosmetics. A very wide range of materials is described. In Example 45 there is described a polymer containing a unit of the formula

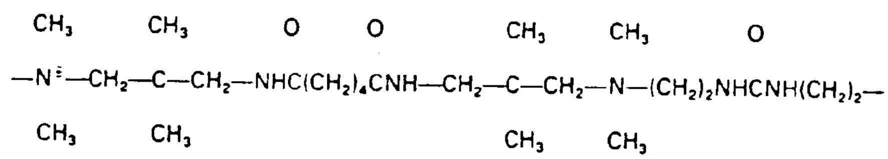

No cosmetic formulation using this compound was reported, however.

Summary of the invention and description of the preferred embodiments

The compounds of the present invention are conditioners and softeners having outstanding properties for use in hair and skin care compositions such as shampoos, soaps and lotions, and for use in scale inhibition and as flocculants.

The present compounds are of the general Formula (I):

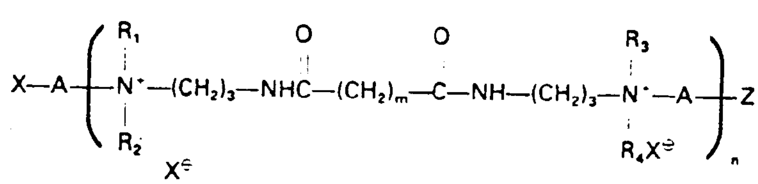

wherein $R_1$, $R_2$, $R_3$ and $R_4$ are methyl;

n is the value required to produce a weight average molecular weight of about 52,500;
m is 4
X is chloro
Z is chloro

and

A is $-CH_2CH_2OCH_2CH_2-$.

The polyquaternary compounds are prepared by condensation of a polyamine and a quaternizing agent as follows:

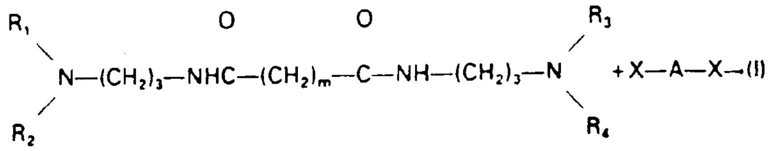

The polyamine in turn is prepared by condensation of two diamino compounds with a dicarboxylic acid. The diamino compounds are of the formula

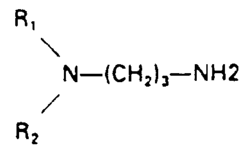

and

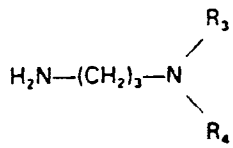

and may be identical, wherein $R_1$ and $R_2$ are identical to $R_3$ and $R_4$.

The polyquaternary ammonium compounds of the invention are generally present in amounts of from about 1 to 10% by weight based on the total cosmetic composition. Shampoos and liquid soaps generally contain about 1 to 10% by weight and lotions generally contain about 0.5 to 5% by weight.

The cosmetic formulations comprise a suitable cosmetic carrier, the composition of which carrier depends on the purpose for which the final composition is intended. Thus, different carriers will be used dependent on whether the composition is to be used as a shampoo, a soap etc. These carriers are as described in the art. They may contain surfactants such as sodium lauryl sulfate, sodium lauryl ether sulfate, sodium lauryl sarcosinate, lauroamphocarboxyglycinate or co-coamphocarboxyglycinate, solvents such as glycol and propylene glycol, proteins, citric acid, coloring agents, preservatives, fragrances, mineral oils, thickeners such as sodium chloride, PEG 6000 distearate, PEG 80 sorbitan laurate, cocamide DEA, lauramide DEA or hydroxypropyl methylcellulose, vitamins, silicones, emollients such as lanolin, isopropyl palmitate, isopropyl myristate or petrolatum and viscosity builders such as carrageenan, carbomers or cocamidopropyl hydroxy sultaine. Where appropriate, CTFA designation is provided herein above and hereinafter.

Example I

A. To a reaction flask was charged 438 grams 13 moles) of adipic acid. After heating to about 55°C, 612 grams (6 moles) of dimethylamino-propylamine was added in about five minutes. During this addition the temperature rose to 101°C. The reaction mixture was healed to 165-170°C in about 3 to 4 hours. Distillation started at about 147°C. After a temperature of 165°C was reached, 62 grams of dimethylamino-propylamine (DMAPA) was added over a half hour period. The reaction mixture was then heated to 180 to 185°C in about two hours. After a temperature of 180°C was reached, 62 grams of DMAPA was added in about a half hour and the temperature maintained at 180-185°C for 1/2 hour. Vacuum was applied to remove water and excess DMAPA at a gradual increase to 5-10 mm. The vacuum of 5-10 mm was maintained for an hour at 180-185°C. The reaction mixture was cooled to 100°C and vacuum decreased to recover Adipic Condensate.

The above reaction was repealed by replacing adipic acid (1) with equal molar amounts of azelaic acid and (2) with equal molar amounts of dimer acid $HOOC\text{-}(CH_2)_{34}\text{-}COOH$.

B. To a two liter reaction flask was charged 282.73 grams (0.88 moles) of Adipic Condensate formed under Example A above and 152.24 g water and the mixture was healed to 90°C. Over four hours 122.28 g (0.855 moles) dichloroethylether was added while a temperature of 90-100°C was maintained. After the addition

was completed, the reaction mass was heated to 100°C and held for 5 hours at about 100°C. The reaction was found to be 99.7% completed by chloride ion analysis.

115.8 g Water was added to the amber colored material and the mixture heated to distill 51.4 g distillate to remove traces of unreacted dichloroethylether. The final product was a clear amber colored liquid. The pH of a 10% aqueous solution was 8.32. The solids content was 68.2% by Karl Fischer (31.8% water) determination (as described by the American Oil Chemistry Society). The compound was found to have a value for n of about 115, determined from an intrinsic viscosity average molecular weight $\overline{M}_w$ of 52,500 and an $\overline{M}_n$ (number average molecular weight) of 45,800.

## Application Examples

Two polyquaternaries according to the invention were evaluated as conditioners in hair and skin care products.

Four formulations were prepared having the following ingredients:

| | Conditioning shampoo A | B | D |
|---|---|---|---|
| Sodium lauryl ether sulfate | 21.0 | 21.0 | 21.0 |
| Mirataine® CB | 15.0 | 15.0 | 15.0 |
| Miranol® C2M conc. | 10.5 | 10.5 | 10.5 |
| Mirapol® A-15 | 2.1 | — | — |
| Product of Example IB | — | 2.1 | — |
| Water | 51.4 | 51.4 | 53.5 |

Each formulation was adjusted to a pH of 7.0.

Mirataine CB is cocamidopropyl betaine and is a high foamer and viscosity builder.

Miranol C2M conc. is a viscous clear aqueous amphoteric surfactant solution derived from coconut fatty acids, specifically a dicarboxymethylaled derivative of cocoimidazoline.

Mirapol A-15 is a hair and skin conditioner as described in U.S. Patent No. 4,157,388.

The four formulations were evaluated by testing of hair swatches as to wet comb and dry comb results, antistatic effect, softness, shine etc. Formulation B was found to perform better than A as regards conditioning ability, that is manageability and wet and dry comb out.

| | Conditioning shampoo A | B | D |
|---|---|---|---|
| Alpha olefin sulfonate (A.O.S.) (40%) | 15.0 | 15.0 | 15.0 |
| Miranol® 2MCAS-mod. | 15.0 | 15.0 | 15.0 |
| Mirataine® CBS | 15.0 | 15.0 | 15.0 |
| Mirapol® A-15 | 2.1 | — | — |
| Example IB | — | 2.1 | — |
| Polysorbate 20 (Tween® 20, ICI Americas Inc.) | 2.0 | 2.0 | 2.0 |
| Water | 50.9 | 50.9 | 53.0 |

Miranol 2MCAS-mod. is a mixture of the lauryl sulfate and laureth-3 sulfate salts of a dicarboxymethylated derivative of a cocoimidazoline having one-eye irrating properties.

Mirataine CBS is cocamidopropyl hydroxysultaine.

Each formulation was adjusted to a pH of 7.0.

The same evaluation as above was performed on hair swatches and again B was best.

| Soft soap | A | B | D | E |
|---|---|---|---|---|
| A.O.S. (40%) | 15.0 | 15.0 | 15.0 | 15.0 |
| Cedepal® TD 404M | 10.0 | 10.0 | 10.0 | 10.0 |
| Mirataine® CB | 20.0 | 20.0 | 20.0 | 20.0 |
| Cocamide DEA (super amide GR, Onyx Chem. Co.) | 2.5 | 2.5 | 2.5 | 2.5 |
| Mirapol® A-15 | 2.1 | — | — | — |
| Example IB | — | 2.1 | — | — |
| Polyquaternium 7 Merquat 550 (Merck Chem. Co.) | — | — | 2.1 | — |
| Glycol stearate (Cerasynt, IP Van Dyk & Co.) | 1.0 | 1.0 | 1.0 | 1.0 |
| Glydant® (DMDM hydantoin, Glycochemicals) | 0.2 | 0.2 | 0.2 | 0.2 |
| NaCl | 1.0 | 1.0 | 1.0 | 1.0 |
| Water | 48.2 | 48.2 | 48.2 | 50.3 |

The pH of each formulation was adjusted to 7.0.

Cedepal TD 404 M is sodium trideceth sulfate.

Super Amide GR is a cocodiethanolamide.

Merquat 550 is a cationic polymer.

Glydant is a preservative.

A panel of five people after washing their hands with the five formulations chose B as the best in afterfeel and skin conditioning, B was slightly better than D.

| Sheen activator | A | B | D |
|---|---|---|---|
| Water | 81.4 | 81.4 | 83.5 |
| Mirapol® A-15 | 2.1 | — | — |
| Example IB | — | 2.1 | — |
| Glycerine | 10.0 | 10.0 | 10.0 |
| Peptein® 2000 (Hormel) | 5.0 | 5.0 | 5.0 |
| Phenoxyethanol (Emeressence 1160, Emery Industries, Inc.) | 1.0 | 1.0 | 1.0 |
| Cellosize® QP 4400 (Union Carbide) | 0.5 | 0.5 | 0.5 |

The formulations were adjusted to a pH of 5.5.

The four formulations above were evaluated as sheen activators and pre-style lotions to determine enhancement in shine and setting properties.

A panel of five people chose B as best.

| Skin moisturizer/conditioner | A | B | D |
|---|---|---|---|
| Self emulsifying wax | 7.5 | 7.5 | 7.5 |
| PEG-400 Distearate | 2.5 | 2.5 | 2.5 |
| Stearic acid | 1.0 | 1.0 | 1.0 |
| Cetyl alcohol | 1.0 | 1.0 | 1.0 |
| Mirapol A-15 | 2.1 | — | — |
| Example IB | — | 2.1 | — |
| Merquat 100 | — | — | 2.1 |
| Propylene glycol | 3.5 | 3.5 | 3.5 |
| Methyl paraben | 0.1 | 0.1 | 0.1 |
| Propyl paraben | 0.2 | 0.2 | 0.2 |
| Water | 82.1 | 82.1 | 82.1 |

Prototype B was chosen as the best in skin Softening and Conditioning. The new polyquaternary ammonium compounds were evaluated as antistats in hair care products.

Eight formulations were prepared having the following ingredients:

| | D | E | F | G | H | Control (untreated) |
|---|---|---|---|---|---|---|
| Sodium lauryl ether sulfate (Maprofix ES) | 21.0 | 21.0 | 21.0 | 21.0 | 21.0 | — |
| Mirataine CB | 15.0 | 15.0 | 15.0 | 15.0 | 15.0 | — |
| Miranol C2M | 10.5 | 10.5 | 10.5 | 10.5 | 10.5 | — |
| Example IB* | 1.7 | — | — | — | — | — |
| Mirapol A-15* | — | 1.5 | — | — | — | — |
| Merquat 100* | — | — | 2.5 | — | — | — |
| Polymer JR-400* (3% solution) | — | — | — | 33.3 | — | — |
| Water | 51.8 | 52.0 | 51.0 | 20.2 | 53.5 | — |
| $X10^{-7}$ Coulombs | 1.4 | 7.2 | 7.1 | 8.4 | 9.2 | 4.7 |

* Used 1% on active basis.

Hair swatches were soaked for half hour in a 25% solution of the above formulas and rinsed with 40°C water for one minute. The hair swatches were dried overnight at ambient temperature and the antistatic property was evaluated by measuring electrostatic charges with an Electrometer (Keithley Model 610C). Formulation (D) exhibited the lowest static charge of the eight formulas. There was no significant difference in values obtained for the other formulations.

## Claims

1. Polyquaternary ammonium compounds of the formula:

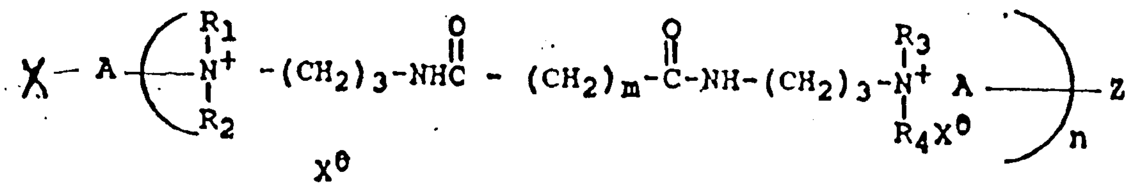

wherein $R_1$, $R_2$, $R_3$ and $R_4$ are methyl;
n is the value required to produce a weight average molecular weight of about 52, 500;
m is 4;
X is chloro;
Z is chloro; and
A is $-CH_2CH_2-O-CH_2CH_2-$.

2. A cosmetic composition which comprises a suitable cosmetic carrier and a quaternary ammonium compound of the formula:

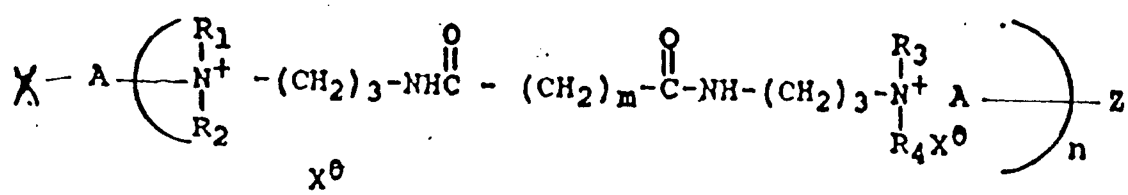

wherein $R_1$, $R_2$, $R_3$ and $R_4$ are methyl;
n is the value required to produce a weight average molecular weight of about 52,500;
m is 4;
X is chloro;
Z is chloro; and
A is $-CH_2CH_2-O-CH_2CH_2-$.

3. A composition according to claim 2, wherein the cosmetic carrier is one for use in hair formulations.

4. A composition according to claim 3, wherein the hair formulation is a shampoo.

5. A composition according to claim 2 or 3, wherein the cosmetic carrier is one for use in skin care products.

6. A composition according to claim 5, wherein the skin care product is a soap.

## Patentansprüche

1. Polyquaternäre Ammoniumverbindungen der Formel:

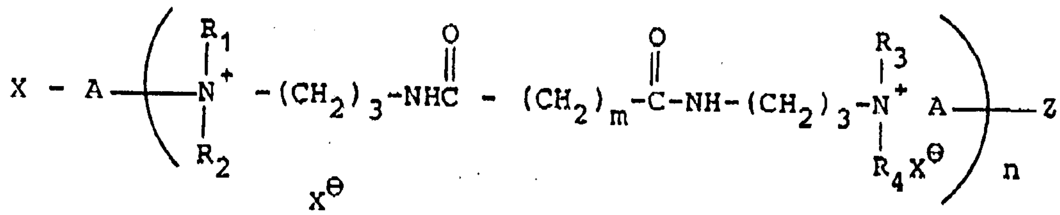

worin sind:

$R_1$, $R_2$, $R_3$ und $R_4$ Methyl;

n .... ein zum Erzeugen eines gewichteten mittleren Molekulargewichts von etwa 52.000 erforderlicher Wert;

m .... 4;

X .... Chlor und

A .... $CH_2CH_2$-O-$CH_2CH_2$-.

2. Kosmetische Zusammensetzung, welche einen geeigneten kosmetischen Träger und eine quaternäre Ammoniumverbindung der Formel :

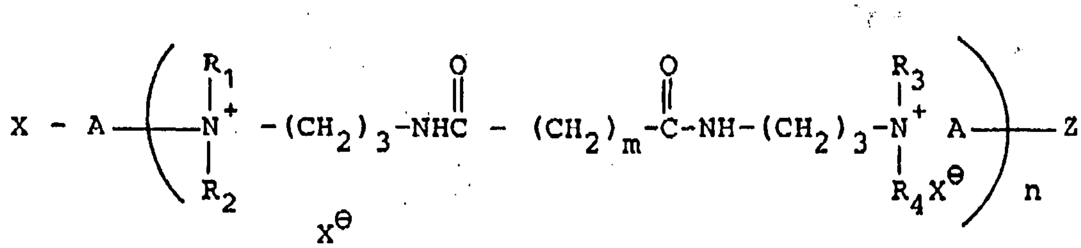

umfaßt, worin sind:

$R_1$, $R_2$ , $R_3$ und $R_4$ Methyl;

n .... ein zum Erzeugen eines gewichteten mittleren Molekulargewichts von etwa 52.500 erforderlicher Wert;

m .... 4;

X .... Chlor;

Z .... Chlor und

A .... -$CH_2CH_2$-O-$CH_2CH_2$-.

3. Zusammensetzung nach Anspruch 2, in welcher der kosmetische Träger ein für Haarpräparate gebräuchlicher Träger ist.

4. Zusammensetzung nach Anspruch 3, in welcher das Haarpräparat ein Schampoon ist.

5. Zusammensetzung nach Anspruch 2 oder 3, in welcher der kosmetische Träger ein für Hautpflegemittel gebräuchlicher Träger ist.

6. Zusammensetzung nach Anspruch 5, in welcher das Hautpflegemittel eine Seife ist.

## Revendications

1. Composés de poly(ammonium quaternaire) de formule :

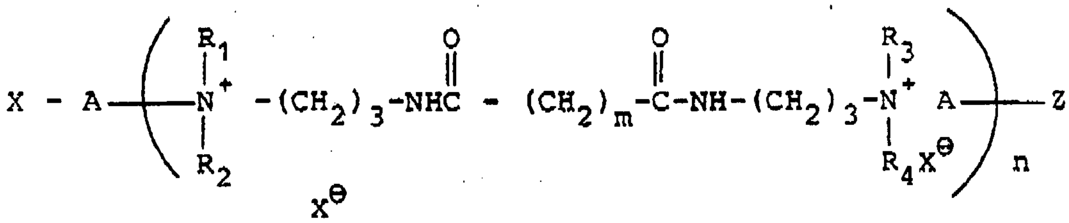

dans laquelle $R_1$, $R_2$, $R_3$ et $R_4$ sont un groupe méthyle,

n est la valeur requise pour obtenir un poids moléculaire moyen en poids d'environ 52.500 ;
m est 4 ;
X est un chlore ;
Z est un chlore ; et
A est $-CH_2CH_2-O-CH_2CH_2-$.

2. Composition cosmétique qui comprend un véhicule cosmétique convenable et un composé d'ammonium quaternaire de formule :

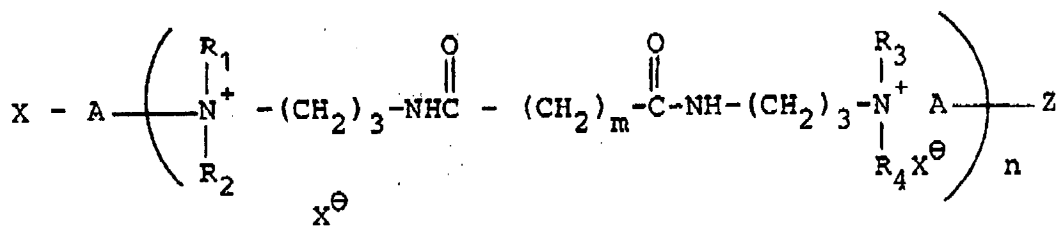

dans laquelle $R_1$, $R_2$, $R_3$ et $R_4$ sont un groupe méthyle,

n est la valeur requise pour obtenir un poids moléculaire moyen en poids d'environ 52.500 ;
m est 4 ;
X est un chlore ;
Z est un chlore ; et
A est $-CH_2CH_2-O-CH_2CH_2-$.

3. Composition selon la revendication 2, dans laquelle le véhicule cosmétique est un véhicule utilisable dans les formulations pour cheveux.

4. Composition selon la revendication 3, dans laquelle la formulation pour cheveux est un schampooing.

5. Composition selon l'une quelconque des revendications 2 ou 3, dans laquelle le véhicule cosmétique est un véhicule utilisable dans les produits de soins de la peau.

6. Composition selon la revendication 5, dans laquelle le produit de soins de la peau est un savon.